# NEUE EUROPÄISCHE PATENTSCHRIFT

(11) **EP 1 268 379 B2**
(45) Veröffentlichungstag und Bekanntmachung des Hinweises auf die Entscheidung über den Einspruch: **17.06.2009**
(45) Hinweis auf die Patenterteilung: 03.03.2004
(21) Anmeldenummer: 01921334.7
(22) Anmeldetag: 14.03.2001
(51) Int. Cl.: C07C 39/16, C07C 37/84

(54) **VERFAHREN ZUR HERSTELLUNG VON BISPHENOLEN**
METHOD FOR PRODUCING BISPHENOLS
PROCEDE DE PREPARATION DE BISPHENOLS

(30) Priorität: 27.03.2000 DE 10015014
(43) Veröffentlichungstag der Anmeldung: 02.01.2003
(73) Patentinhaber: Bayer MaterialScience AG, 51368 Leverkusen (DE)
(72) Erfinder: NEUMANN, Rainer, 47803 Krefeld (DE); LANZE, Rolf, 47804 Krefeld (DE); HEYDENREICH, Frieder, 40593 Düsseldorf (DE); BÖDIGER, Michael, League City, TX 77573 (US); PREIN, Michael, B-2930 Brasschaat (BE)
(86) Internationale Anmeldenummer: PCT/EP2001/002845
(87) Internationale Veröffentlichungsnummer: WO 2001/072677

(56) Entgegenhaltungen:
- EP-A- 0 671 377
- US-A- 5 723 688
- WOLFGANG WÖHLKE, GÜNTER HOFMANN: 'Zum Beispiel Bisphenol A-Phenol' CHEMISCHE INDUSTRIE Juni 1991, Seiten 62 - 70

## Beschreibung

Die vorliegende Erfindung betrifft Kristalle aus einem Addukt aus einem Bisphenol und einem Phenol, ein Verfahren zur Herstellung dieser Kristalle und ein Verfahren zur Herstellung von Bisphenolen.

Bis(4-hydroxyaryl)alkane, im Folgenden Bisphenole genannt, sind als Ausgangsstoffe oder als Zwischenprodukte zur Herstellung einer Vielzahl kommerzieller Produkt von Bedeutung. Bisphenole können durch die Kondensation von Phenolen und Carbonylverbindungen hergestellt werden. Dabei können substituierte Phenole oder unsubstituiertes Phenol verwendet werden.

Von besonderer technischer Bedeutung ist das Kondensationsprodukt aus der Reaktion zwischen Phenol und Aceton, 2,2-Bis(4-hydroxyphenyl)propan (Bisphenol A, BPA). BPA dient als Ausgangstoff zur Herstellung verschiedenartiger polymerer Werkstoffe wie beispielsweise Polyarylate, Polyetherimide, Polysulfone und modifizierter Phenol-Formaldehydharze. Bevorzugte Anwendungsgebiete liegen in der Herstellung von Epoxyharzen und Polycarbonaten.

Verfahren zur Herstellung von Bisphenolen durch sauerkatalysierte Umsetzung von Phenolen mit Carbonylverbindungen sind beispielsweise bekannt aus US-A-2 775 620 und aus EP-A-0 342 758. EP-A-0 671 337 offenbart ein Verfahren zur Herstellung von Kristallen von Addukten von p, p-Bisphenol-A und Phenol.

Bisphenole allgemeiner Struktur können nach Verfahren hergestellt werden, die den Verfahren zur Herstellung von BPA analog sind.

Die Herstellung der Bisphenole kann über ein Addukt aus Bisphenol und Phenol als Zwischenstufe erfolgen.

Der vorliegenden Erfindung liegt die Aufgabe zugrunde, ein gegenüber dem Stand der Technik verbessertes Verfahren zur Herstellung von Bisphenolen bereitzustellen. Das erfindungsgemäße Verfahren soll insbesondere Bisphenol von hoher Reinheit zugänglich machen.

Diese Aufgabe wird gelöst durch ein Verfahren zur Herstellung von Kristallen eines Adduktes aus einem Bisphenol und einem Phenol umfassend
a) die Bereitstellung einer Lösung enthaltend Bisphenol und Phenol und
b) die kontinuierliche Durchführung der Kristallisation in einer oder mehrerer Kristallisationsvorrichtungen. welche ein Kristallisationsgefäß, eine Umwälzpumpe und einen Kühler enthalten.
wobei die Verweilzeit der zu kristallisierenden Flüssigkeit in den Kristallisationsgefäßen 2 bis 12 Stunden beträgt und
wobei die Kristallisation in 1 bis 5 Stufen jeweils bestehend aus den genannten Kristallisationsvorrichtungen durchgeführt wird und
wobei die Temperatur im Kristallisationsgefäß der letzten Kristallisationsstufe 40 bis 70°C beträgt und
wobei der Gehalt an Bisphenol im Zufluss der ersten Kristallisationsstufe 15 bis 40 Gew.-%, beträgt und wobei im Austritt der letzten Kristallisationsstufe der Gehalt an Bisphenol in gelöster Form in der Mutterlauge 10 bis 20 Gew.-% beträgt und der auskristallisierte Feststoffanteil an Kristallen des Adduktes aus einem Bisphenol und einem Phenol 20 bis 30 Gew.-% beträgt, dadurch gekennzeichnet, dass die spezifische Pumpenenergie maximal 150 Watt/m³ Suspension beträgt und die Umwälzkühler von oben nach unten mit einer Geschwindigkeit von 1 bis 5 m/s durchströmt werden.

Dieses Verfahren ist demgemäß Gegenstand der vorliegenden Erfindung.

Weiterhin wird die erfindungsgemäße Aufgabe gelöst durch ein Verfahren zur Herstellung eines Bisphenoles umfassend die Herstellung des Adduktes aus einem Bisphenol und einem Phenol nach dem erfindungsgemäßen Verfahren und die Gewinnung des Bisphenoles aus dem Addukt aus dem Bisphenol und dem Phenol.

Auch dieses Verfahren ist demgemäß Gegenstand der vorliegenden Erfindung.

Das erfindungsgemäße Verfahren wird so durchgeführt, dass die Temperatur im Kristallisationsgefäß der letzten Kristallisationsstufe 40 bis 70°C, insbesondere 40 bis 50°C, insbesondere 40 bis 43°C beträgt.

Das erfindungsgemäße Verfahren wird so durchgeführt, dass der Gehalt an Bisphenol im Zufluss der ersten Kristallisationsstufe 15 bis 40 Gew.-%, insbesondere 15 bis 35 Gew.-%, insbesondere 25 bis 35 Gew.-%, beträgt.

Durch das erfindungsgemäße Verfahren zur Herstellung von Kristallen aus Addukten aus Bisphenolen und Phenolen werden diese Kristalle in einer Form und Reinheit zugänglich wie sie aus dem Stand der Technik nicht bekannt ist.

Das erfindungsgemäße Verfahren zur Herstellung von Kristallen aus Addukten aus Bisphenolen und Phenolen hat zahlreiche Vorteile. Es werden Kristalle erhalten, die nach Filtration und Entfernung des Phenols ein Bisphenol derart hoher Qualität erzeugen, dass dieses ohne weitere Aufreinigung für hochwertige Folgeprodukte, beispielsweise Polycarbonate, Epoxidharze, Formaldehydharze u.a., eingesetzt werden kann.

Das erfindungsgemäße Verfahren zur Herstellung von Kristallen aus Addukten aus Bisphenolen und Phenolen und damit das erfindungsgemäße Verfahren zur Herstellung von Bisphenolen hat weiterhin den folgenden Vorteil. Es liefert die Addukte aus Bisphenolen und Phenolen und das Bisphenol in einer so hohen Reinheit, dass die üblicherweise notwendigen weiteren Aufarbeitungsschritte zur Herstellung eines für hochwertige Polycarbonate geeigneten Bisphenols vermieden werden können. Die genannten üblicherweise notwendigen weiteren Aufarbeitungsschritte sind beispielsweise zusätzliche Kristallisationsschritte oder zusätzliche Destillationsschritte.

Die erfindungsgemäß hergestellten Kristalle aus dem Addukt aus einem Bisphenol und einem Phenol haben zahlreiche Vorteile. Sie sind gut filtrierbar und weisen eine hohe Reinheit auf. Sie weisen einen geringen Gehalt an eingeschlossenen Verunreinigungen auf. Daher wird bei der Abtrennung der Kristalle durch Filtration das Addukt aus einem Bisphenol und einem Phenol in hoher Reinheit zugänglich.

Wird aus den erfindungsgemäß hergestellten Kristallen aus dem Addukt aus einem Bisphenol und einem Phenol das Bisphenol gewonnen, so wird dadurch ein neues vorteilhaftes Verfahren zur Herstellung von Bisphenolen bereitgestellt.

Die vorliegende Erfindung hat zahlreiche Vorteile. Das erfindungsgemäße Verfahren ist einfach und damit kostengünstig. Zusätzliche Aufreinigungsstufen sind nicht erforderlich. Die erfindungsgemäß hergestellten Produkte sind gekennzeichnet durch hohe Qualität, die sich ausdrückt in einer niedrigen Farbzahl, einer hohen Lagerstabilität und einer hohen Thermostabilität.

Erfindungsgemäß können beliebige Bisphenole verwendet werden.
Das erfindungsgemäß bevorzugte Bisphenol ist Bisphenol A.

Erfindungsgemäß können beliebige Phenole verwendet werden.
Das erfindungsgemäß bevorzugte Phenol ist unsubstituiertes Phenol.

Das erfindungsgemäße Verfahren zur Herstellung von Kristallen aus dem Addukt aus einem Bisphenol und einem Phenol ist bevorzugt einstufig bis fünfstufig, besonders bevorzugt einstufig bis dreistufig und ganz besonders bevorzugt zweistufig. Dabei wird in der ersten Stufe bevorzugt bei Temperaturen von weniger als 100°C. bevorzugt weniger als 70°C, und in der letzten Stufe bevorzugt bei Temperaturen unterhalb von 70°C. besonders bevorzugt unterhalb von 50°C, ganz besonders bevorzugt unterhalb von 43°C gearbeitet.

Beim erfindungsgemäßen Verfahren zur Herstellung von Kristallen aus dem Addukt aus Bisphenol und Phenol findet bevorzugt eine Umwälzkristallisation statt. Dabei wird bevorzugt mit einer 20-fachen bis 40-fachen, besonders bevorzugt mit einer 25-fachen bis 35-fachen, insbesondere mit einer 30-fachen Umwälzrate, bezogen auf den Durchsatz je Kristallerstufe gearbeitet. Unter einer Kristallerstufe ist erfindungsgemäß eine Kristallisationsvorrichtung, welche ein Kristallisationsgefäß, eine Umwälzpumpe und ein Kühler enthält, zu verstehen. Die Umwälzrate ist definiert als die pro Zeiteinheit durch die Umwälzpumpe geförderte Menge dividiert durch die Prozeiteinheit der Kristallisation insgesamt zugeführte Menge.

Beim erfindungsgemäßen Verfahren zur Herstellung von Kristallen aus dem Addukt aus einem Bisphenol und einem Phenol wird bevorzugt so gearbeitet, dass der Umwälzstrom dem Kristaller am Fuß des Kristallers tangential zugeführt und mittig am Kopf des Kristallers entnommen wird.

Beim erfindungsgemäßen Verfahren zur Herstellung von Kristallen aus dem Addukt aus einem Bisphenol und einem Phenol wird bevorzugt so gearbeitet, dass die Reaktionsmischung vor der Kristallisation auf Temperaturen unterhalb von 80°C, besonders bevorzugt auf Temperaturen von 75°C, insbesondere 70°C abgekühlt wird.

Beim erfindungsgemäßen Verfahren zur Herstellung von Kristallen aus dem Addukt aus einem Bisphenol und einem Phenol wird die Reaktionsmischung aus der das Addukt kristallisiert wird, bevorzugt unmittelbar vor dem Kristaller in den Umwälzstrom eingemischt.

Beim erfindungsgemäßen Verfahren zur Herstellung von Kristallen aus dem Addukt aus einem Bisphenol und einem Phenol wird bevorzugt so gearbeitet, dass die Aufstromgeschwindigkeit im Kristallisationsgefäß 0,5 bis 4, besonders bevorzugt 2 bis 3, ganz besonders bevorzugt ca. 3 m pro Minute beträgt. Die Aufstromgeschwindigkeit ist dabei die mittlere Geschwindigkeit der Flüssigkeit im Kristallisationsgefäß von unten nach oben.

Beim erfindungsgemäßen Verfahren zur Herstellung von Kristallen aus dem Addukt aus einem Bisphenol und einem Phenol ist es bevorzugt, dass das Kristallisationsgefäß flüssigkeitsgefüllt betrieben werden.

Beim erfindungsgemäßen Verfahren zur Herstellung von Kristallen aus dem Addukt aus einem Bisphenol und einem Phenol ist es bevorzugt, dass oberhalb des Kristallisationsgefäßes eine Umwälzpumpe installiert ist.

Beim erfindungsgemäßen Verfahren zur Herstellung von Kristallen aus dem Addukt aus einem Bisphenol und einem Phenol wird die Suspension aus Reaktionsmischung und darin enthaltenen Adduktkristallen mit einer spezifischen Pumpenergie von maximal 150 Watt/m³ Suspension, bevorzugt von maximal 100 Watt/m³ Suspension umgewälzt.

Beim erfindungsgemäßen Verfahren zur Herstellung von Kristallen aus dem Addukt aus einem Bisphenol und einem Phenol werden die Umwälzkühler von oben nach unten mit einer Geschwindigkeit von 1 bis 5 m/sec., bevorzugt 2 bis 4 m/sec., besonders bevorzugt von 2,8 bis 3,2 m/sec, durchströmt.

Beim erfindungsgemäßen Verfahren zur Herstellung von Kristallen aus dem Addukt aus einem Bisphenol und einem Phenol ist es bevorzugt, dass die Umwälzkühler elektropolierte Rohre enthalten, in denen die Suspension umgewälzt wird. Dabei haben diese elektropolierten Rohre eine Rauigkeit der Oberfläche die durch die Suspension berührt wird von bevorzugt weniger als 1,5 µm, besonders bevorzugt weniger als 1 µm. Dies hat den Vorteil, dass lange Standzeiten der Umwälzkühler erreicht werden, da nur geringe Ablagerungen an den Oberflächen erfolgen.

Beim erfindungsgemäßen Verfahren zur Herstellung von Kristallen aus dem Addukt aus einem Bisphenol und einem Phenol ist es bevorzugt, dass die Umwälzkühler mit temperiertem Warmwasser als Kühlmedium betrieben werden. Hierbei ist es insbesondere bevorzugt, dass die Temperaturdifferenz zwischen temperiertem Warmwasser und zu kühlender Suspension 2 bis 6 K, ganz besonders bevorzugt 3 bis 4 K. beträgt.

Beim erfindungsgemäßen Verfahren zur Herstellung von Kristallen aus dem Addukt aus einem Bisphenol und einem Phenol ist es bevorzugt, dass die Innenfläche der suspensionsführenden Rohre der Umwälzkühler in regelmäßigen Zeitabständen von bevorzugt 40 Tagen durch schnelles Aufheizen auf ca. 80°C von Belägen befreit werden. Diese Beläge können beispielsweise aus Bisphenol und aus Addukt aus Bisphenol und Phenol bestehen.

Beim erfindungsgemäßen Verfahren zur Herstellung von Kristallen aus dem Addukt aus einem Bisphenol und einem Phenol ist es bevorzugt, dass der Kristaller und die zugehörigen Peripheriegeräte in regelmäßigen Zeitabständen von bevorzugt 40 bis 300 Tagen durch Aufheizung auf bevorzugt 80°C gereinigt werden, wobei der Umwälzkreislauf weiter betrieben wird.

Das bevorzugte erfindungsgemäße Verfahren zur Herstellung von Kristallen aus dem Addukt aus einem Bisphenol und einem Phenol ist die Suspensionskristallisation, bei der die Kristalle des Adduktes aus Bisphenol und Phenol durch Abkühlung der Reaktionsmischung erfolgt.

Das erfindungsgemäß bevorzugte Verfahren zur Herstellung von BPA beruht auf der säurekatalysierten Umsetzung von Phenol mit Aceton, wobei bevorzugt ein Mengenverhältnis Phenol:Aceton von größer als 5 : 1 in der Reaktion eingestellt wird. Als saure Katalysatoren können homogene wie auch heterogene Brönstedsäuren oder Lewissäuren genutzt werden, so beispielsweise starke Mineralsäuren wie Salzsäure oder Schwefelsäure. Bevorzugt kommen gelförmige oder makroporöse sulfonierte vernetzte Polystyrolharze (saure Ionentauscher) zum Einsatz. Die nachfolgenden Ausführungen beziehen sich auf ein Verfahren zur Herstellung unter Nutzung von sauren Ionentauschern als Katalysatoren.

Zur Erzielung hoher Selektivitäten kann die Umsetzung von Phenol mit Aceton in Gegenwart geeigneter Mercaptoverbindungen als Cokatalysatoren durchgeführt werden. Diese können entweder homogen in der Reaktionslösung gelöst sein oder über ionische oder kovalente Bindungen an der sulfonierten Poylstyrolmatrix fixiert werden. Die Reaktionseinheit ist bevorzugt ein Schichtbett oder Wirbelbett, die aufoder abwärts durchflossen werden, oder eine Kolonne nach Art einer Reaktivdestillationskolonne.

Bei der Umsetzung von Phenol mit Aceton in Gegenwart saurer Katalysatoren und Mercaptoverbindungen als Cokatalysatoren entsteht eine Produktmischung, die neben nicht umgesetztem Phenol und gegebenenfalls Aceton in erster Linie BPA und Wasser enthält. Daneben treten in geringen Mengen typische Nebenprodukte der Kondensationreaktion auf, so beispielsweise 2-(4-hydroxyphenyl)-2-(2-hydroxyphenyl)propan (o,p-BPA), substituierte Indene, Hydroxyphenyl-indanole, Hydroxyphenyl-chromane, substituierte Xanthene und höher kondensierte Verbindungen mit drei oder mehr Phenylringen im Molekülgerüst.

Die genannten Nebenprodukte wie auch Wasser, Phenol und Aceton können die Eignung von BPA zur Herstellung von Polymeren beeinträchtigen und werden bevorzugt durch geeignete Verfahren abgetrennt werden. Insbesondere zur Herstellung von Polycarbonat werden üblicherweise hohe Reinheitsanforderungen an den Rohstoff BPA gestellt.

Die Aufarbeitung und Reinigung von BPA erfolgt üblicherweise durch eine mehrstufige Kaskade von geeigneten Reinigungsverfahren wie beispielsweise Suspensionskristallisation, Schmelzekristallisation, Destillation und Desorption. In einer bevorzugten Ausführungsform erfolgt die Abtrennung von BPA aus der Reaktionsmischung in Form eines etwa äquimolaren kristallinen Addukts mit Phenol durch Abkühlen der Reaktionsmischung unter Auskristallisieren des BPA/Phenol-Addukts. Die Kristallisation erfolgt bevorzugt als Suspensionskristallisation. Unter Suspensionskristallisation versteht man die Kristallisation aus einer Flüssigkeit durch Abkühlung, wobei die Kristalle mit der Flüssigkeit eine Suspension bilden. Die BPA/Phenol-Adduktkristalle werden anschließend durch eine geeignete Apparatur zur Fest-Flüssigtrennung wie Drehfilter oder Zentrifugen von der Flüssigphase abgetrennt und erforderlichenfalls der weiteren Reinigung zugeführt. So erhaltene Adduktkristalle weisen typischerweise eine Reinheit von größer als 99 Gew.-% BPA bezogen auf die Nebenkomponenten bei einem Phenolanteil von ca. 40 Gew.-% auf. Durch Waschen mit geeigneten Lösungen, die typischerweise eine oder mehrere Komponenten aus der Gruppe Aceton, Wasser, Phenol, BPA und Nebenkomponenten enthalten, können die Adduktkristalle von oberflächlich anhaftenden Verunreinigungen befreit werden.

Der bei der Fest-Flüssigtrennung anfallende Flüssigstrom (Mutterlauge) enthält Phenol, BPA, bei der Reaktion entstandenes Wasser, nicht umgesetztes Aceton und ist angereichert an den bei der BPA-Herstellung typischerweise anfallenden Nebenkomponenten. Dieser Mutterlaugenstrom wird in einer bevorzugten Ausführungsform in die Reaktionseinheit zurückgeführt. Um die katalytische Aktivität der sauren Ionentauscher aufrecht zu erhalten wird zuvor entstandenes Wasser bevorzugt durch Destillation entfernt, wobei auch gegebenenfalls noch vorhandenes Aceton aus der Mutterlauge entfernt wird. Der so erhaltene entwässerte Reaktionsstrom wird bevorzugt um Phenol und Aceton ergänzt und in die Reaktionseinheit zurückgeführt. Alternativ können auch vor Durchführung der Suspensionskristallisation des BPA-Phenol-Addukts Wasser und Aceton ganz oder teilweise destillativ entfernt werden. Bei den genannten Destillationsschritten kann auch eine Teilmenge des in der Reaktionslösung vorhandenen Phenols destillativ abgetrennt werden.

Bei einer derartigen Kreislauffahrweise tritt als Problem auf, dass Nebenprodukte der BPA-Herstellung im Kreislaufstrom angereichert werden und zur Desaktivierung des Katalysatorsystem führen können. Um eine übermäßige Anreicherung von Nebenkomponenten im Kreislaufstrom zu vermeiden, wird bevorzugt eine Teilmenge des Kreislaufstroms - gegebenenfalls nach teilweiser oder vollständiger destillativer Rückgewinnung von Phenol - aus der Prozesskette als BPA-Harz ausgeschleust.

Außerdem hat es sich als vorteilhaft erwiesen einen Teil oder die Gesamtmenge des Kreislaufstroms nach der Fest-Flüssigtrennung und vor oder nach der Abtrennung von Wasser und Restaceton über eine mit saurem Ionentauscher befüllte Umlagerungseinheit zu führen. Diese Einheit wird im allgemeinen bei höheren Temperaturen betrieben als die Reaktionseinheit. In dieser Umlagerungseinheit werden unter den vorherrschenden Bedingungen einige der im Kreislaufstrom vorhandenen Nebenkomponenten der BPA-Herstellung zu BPA isomerisiert, so dass die Gesamtausbeute an BPA erhöht werden kann.

Die im Anschluss an die oben beschriebene Suspensionskristallisation der Reaktionslösung und Fest-Flüssig-Trennung erhaltenen BPA-Phenol-Adduktkristalle werden erforderlichenfalls weitergehenden Reinigungsschritten zugeführt, wobei die Abtrennung des überwiegenden Teils des Phenols erzielt wird.

So können die Adduktkristalle beispielsweise aus Phenol, aus organischen Lösungsmitteln, aus Wasser oder aus Mischungen der genannten Verbindungen gemäß einer Suspensionskristallisation umkristallisiert werden. Hierbei kann durch die Wahl geeigneter Lösungsmittel auch das in den Adduktkristallen vorhandene Phenol ganz oder teilweise abgetrennt werden. Das gegebenenfalls nach der Umkristallisation im BPA verbleibende Phenol kann anschließend durch geeignete destillative, desorptive oder extraktive Methoden gänzlich abgetrennt werden.

Alternativ kann auch zunächst Phenol aus den Adduktkristallen entfernt werden. Bevorzugte Methoden hierbei sind Desorption der Schmelze mit heißen Inertgasen, Vakuumdestillation oder eine Kombination der genannten Methoden. Auf diesem Wege ist es möglich aus den Adduktkristallen BPA mit einem Restphenolgehalt von weniger als 100 ppm zu gewinnen. Durch geeignete Reaktionsführung und gegebenenfalls Zugabe von Stabilisatoren kann erreicht werden, dass BPA unter der thermischen Belastung der destillativen oder desorptiven Phenolentfernung nicht in nennenswertem Umfang gespalten wird.

In Abhängigkeit von den Verfahrensbedingungen der Suspensionskristallisation aus der Reaktionslösung und der Durchführung der Fest-Flüssigtrennung und Kristallwäsche ist das nach Abtrennung von Phenol aus den Adduktkristallen erhaltene BPA zur Herstellung von polymeren Werkstoffen geeignet. Insbesondere zur Herstellung hochwertiger Werkstoffe wie Polycarbonat kann es nötig sein, das nach Abtrennung von Phenol erhaltene BPA einer weiteren Reinigungsoperation zuzuführen. Die Endreinigung kann erfolgen durch Suspensionskristallisation aus Wasser oder geeigneten organischen Lösungsmitteln, Schmelzekristallisation in Form einer statischen oder dynamischen Schichtkristallisation, Extraktion mit Wasser, wässrigen neutralen, sauren oder basischen Salzlösungen oder geeigneten organischen Lösungsmitteln oder in Form einer ein- oder mehrstufigen Destillation. Durch die Durchführung der genannten Reinigungsoperationen oder einer geeigneten Kombination derselben ist es möglich, BPA mit einer Reinheit von größer als 99,5 Gew.-% zu erhalten, das zur Herstellung hochwertiger Polymerwerkstoff in besonderer Weise geeignet ist.

Im Folgenden wird eine bevorzugte Ausführungsform der Erfindung anhand einer Abbildung (Fig. 1) erläutert. Die Erfindung ist nicht auf diese bevorzugte Ausführungsform beschränkt.

Fig. 1 stellt eine zweistufige Vorrichtung zur Herstellung von Kristallen aus Addukten aus Bisphenol und Phenol dar. Die Zuführung der Reaktionsmischung, die Bisphenol und Phenol enthält, zur Vorrichtung erfolgt über die Rohrleitung 1. Diese mündet in die Rohrleitung 2, welche den Umwälzstrom aus dem Wärmetauscher 3 in das erste Kristallisationsgefäß 4 führt. Die Zuführung erfolgt dabei am Fuß des Kristallisationsgefäßes tangential. Seitlich am Kopf des Kristallisationsgefäßes wird durch die Rohrleitung 5, die Suspension aus Reaktionsmischung und Kristallen aus Addukt aus Bisphenol und Phenol entnommen und über die Umwälzpumpe 6 dem Wärmetauscher 3 zugeführt. Über die Rohrleitung 7 wird Suspension aus dem ersten Kristallisationsgefäß entnommen und der zweiten Kristallisationsstufe bestehend aus zweitem Kristallisationsgefäß 8, zweitem Wärmetauscher 9, zweiter Umwälzpumpe 10 zugeführt. Schließlich wird die Suspension zur weiteren Aufarbeitung aus dem zweitem Kristallisationsgefäß 8 über die Rohrleitung 11 abgeführt.

Im Folgenden wird die Erfindung anhand eines Beispieles erläutert, ohne auf dieses beschränkt zu sein.

### Beispiel 1 (erfindungsgemäß)

Zur Aufarbeitung und Reinigung von BPA wurde die Reaktionsmischung aus der BPA-Herstellung einer Suspensionskristallisation in einer Vorrichtung gemäß Fig. 1 zugeführt. Hierzu wurde eine 2-stufige Kristallisation, beginnend in der 1. Stufe bei 56°C und der 2. Stufe bei 41°C durchgeführt, wobei der Bisphenolgehalt der zu kristallisierenden Reaktionslösung 30 % betrug. Die Reaktionsmischung wurde unmittelbar vor dem Kristallisationsgefäß mit 70°C in den Umwälzstrom eingemischt und mit einer Aufstromgeschwindigkeit im Kristallisationsgefäß von ca. 3 m/min betrieben.

Die Umwälzkristallisation wurde dabei mit 30-facher Umwälzrate (bezogen auf Durchsatz) je Kristallerstufe betrieben und der Umwälzstrom dem Kristallisationsgefäß am Fuß tangential zugeführt und mittig am Kopf des Kristallisationsgefäßes entnommen.

Die Umwälzkühler wurden von oben nach unten mit einer Geschwindigkeit von 3 m/s durch elektropolierte Rohre (Vorteil: lange Standzeiten der Kristaller-Umwälzkühler durch nur geringe Ablagerungen an den Kühlflächen) (Oberflächenrauhigkeit 1 µm) geführt und die Kühler mit temperiertem Warmwasser betrieben. Die maximale Temperaturdifferenz zur Produktseite betrug 3 bis 4 K.

Die Umwälzpumpe war dabei oberhalb des Kristallisationsgefäßes vor dem Umwälzkühler angeordnet und wurde mit einer spez. Pumpenenergie von max. 100 Watt/m³ Suspension betrieben.

Die fertige Mischkristallsuspension wurde nach einer Gesamtverweilzeit von 4 Stunden seitlich am Kopf des Kristallisationsgefäßes entnommen.

Zur Befreiung von BPA- und BPA/Phenol-Belägen wurden die Innenflächen der Kühlrohre in regelmäßigen Zeitabständen durch eine Schnellaufheizung auf 80°C gereinigt bzw. das gesamte Kristallersystem in regelmäßigen Zeitabständen durch Aufheizung des Produktinhaltes und Weiterbetrieb des Umwälzkreislaufes gereinigt.

Durch diese Art der Kristallisation konnte Bisphenol A-Phenoladduktkristall mit hohen Reinheiten erhalten werden.

Nach Filtration und Phenolabtrennung wurde Bisphenol A mit einer Reinheit von 99,5 % und einer Farbzahl von 20 Hazen erhalten. Die Hazen-Farbzahl wurde visuell durch den Vergleich mit APHA Standard-Colometrie-Lösungen bestimmt. Der Wert ist die Anzahl mg Platin [als Kaliumhexachloroplatinat (IV) mit Cobalt(II)-chloridhexahydrat im Verhältnis 1,246:1 in 1000 ml wässriger Salzsäure gelöst], die in gleicher Schichtdicke die gleiche Farbe wie die Probe aufweist.

Das so erzeugte Bisphenol A kann nach den üblichen Verfahren zu hochreinem Polycarbonat umgesetzt werden. Aus der in Beispiel 1 erhaltenen Bisphenol-A-Probe (Reinheit >99,5 %, Farbzahl 20 Hazen) wurde unter Sauerstoffausschluss durch Hinzufügen von Natriumhydroxid (6,5 % in Wasser) eine 14 %ige wässrige Lösung von Natriumbisphenolat hergestellt. Diese Lösung wurde im Phasengrenzflächenverfahren mit Phosgen und Phenol umgesetzt. Nach Aufarbeitung wurde ein Polycarbonat der relativen Lösungsviskosität 1,297 erhalten. Der YI (Yellowness Index) des Polycarbonats betrug 2,3, die Lichtdurchlässigkeit (Transmission ASTM D 1003) 87,88.

Der Yellowness-Index YI wurde nach ASTM D 1925, die Transmission nach ASTM D 1003 gemessen. Die relative Lösungsviskosität wurde an einer 5 g Polymer/l enthaltenden Lösung in Dichlormethan bei 25°C bestimmt.

### Beispiel 2 (Vergleichsbeispiel)

Zur Aufarbeitung und Reinigung von BPA wurde die Reaktionsmischung aus der BPA-Herstellung einer Suspensionskristallisation in einer Vorrichtung gemäß Fig. 1 zugeführt. Hierzu wurde eine 2-stufige Kristallisation, beginnend in der 1. Stufe bei 56°C und der 2. Stufe bei 41°C durchgeführt, wobei der Bisphenolgehalt der zu kristallisierenden Reaktionslösung 30 % betrug. Die Reaktionsmischung wurde unmittelbar vor dem Kristallisationsgefäß mit 70°C in den Umwälzstrom eingemischt und mit einer Aufstromgeschwindigkeit im Kristallisationsgefäß von ca. 3 m/min betrieben.

Die Umwälzkristallisation wurde dabei mit 5-facher Umwälzrate (bezogen auf Durchsatz) je Kristallerstufe betrieben und der Umwälzstrom dem Kristallisationsgefäß am Fuß tangential zugeführt und mittig am Kopf des Kristallisationsgefäßes entnommen.

Die Umwälzkühler wurden von oben nach unten mit einer Geschwindigkeit von 0,5 m/s durch nicht elektropolierte Rohre geführt und die Kühler mit temperiertem Warmwasser betrieben. Die maximale Temperaturdifferenz zur Produktseite betrug 3 bis 4 K.

Die Umwälzpumpe war dabei oberhalb des Kristallisationsgefäßes vor dem Umwälzkühler angeordnet und wurde mit einer spez. Pumpenenergie von max. 50 Watt/m³ Suspension betrieben.

Die fertige Mischkristallsuspension wurde nach einer Gesamtverweilzeit von 4 Stunden seitlich am Kopf des Kristallisationsgefäßes entnommen.

Zur Befreiung von BPA- und BPA/Phenol-Belägen mussten die Innenflächen der Kühlrohre in kurzfristigen Zeitabständen durch eine Schnellaufheizung auf 80°C gereinigt bzw. das gesamte Kristallersystem in kurzen Zeitabständen (1 x Woche) durch Aufheizung des Produktinhaltes und Weiterbetrieb des Umwälzkreislaufes gereinigt.

Nach Filtration und Phenolabtrennung von den so erhaltenen Bisphenol A-Phenoladduktkristallen wurde Bisphenol A mit einer Reinheit von 99,3 % und einer Farbzahl von 50 Hazen erhalten. Das so erzeugte Bisphenol A kann nach den üblichen Verfahren nicht zu hochreinem Polycarbonat verarbeitet werden.

Aus der in Vergleichsbeispiel 2 erhaltenen Bisphenol-A-Probe (Reinheit 99,3 %, Farbzahl 50 Hazen) wurde unter Sauerstoffausschluss durch Hinzufügen von Natriumhydroxid (6,5 % in Wasser) eine 14 %ige wässrige Lösung von Natriumbisphenolat hergestellt. Diese Lösung wurde im Phasengrenzflächenverfahren mit Phosgen und Phenol umgesetzt. Nach Aufarbeitung wurde ein Polycarbonat der relativen Lösungsviskosität 1,297 erhalten. Der YI (Yellowness Index) des Polycarbonats betrug 3,1, die Lichtdurchlässigkeit (Transmission ASTM D 1003) 87,36 %.

Der Yl wurde bestimmt wie unter Beispiel 1 beschrieben.

## Patentansprüche

1. Verfahren zur Herstellung von Kristallen eines Adduktes aus einem Bisphenol und einem Phenol umfassend
a) die Bereitstellung einer Lösung enthaltend bisphenol und Phenol und
b) die kontinuierliche Durchführung der Kristallisation in einer oder mehreren Kristallisationsvorrichtungen, welche ein Kristallisationsgefäß, eine Umwälzpumpe und einen Kühler enthalten,
wobei die Verweilzeit der zu kristallisierenden Flüssigkeit in dem Kristallisationsgefäßen 2 bis 12 Stunden beträgt und
wobei die Kristallisation in 1 bis 5 Stufen jeweils bestehend aus den genannten Kristallisationsvorrichtungen durchgeführt wird und
wobei die Temperatur im Kristallisationsgefäß der letzten Kristallisationsstufe 40 bis 70°C beträgt und wobei der Gehalt an Bisphenol im Zufluss der ersten Kristallisationsstufe 15 bis 40 Gew.-% beträgt und
wobei im Austritt der letzten Kristallisationsstufe der Gehalt an Bisphenol in gelöster Form in der Mutterlauge 10 bis 20 Gew.-% beträgt und der auskristallisierte Feststoffanteil an Kristallen des Adduktes aus einem Bisphenol und einem Phenol 20 bis 30 Gew.-% beträgt, **dadurch gekennzeichnet, dass** die spezifische Pumpenenergie maximal 150 Watt/m³ Suspension beträgt und die Umwälzkühler von oben nach unten mit einer Geschwindigkeit von 1 bis 5 m/s durchströmt werden.

2. Verfahren gemäß Anspruch 1, wobei das Bisphenol Bisphenol A ist und wobei das Phenol unsubstituiertes Phenol ist.

3. Verfahren zur Herstellung eines Bisphenols umfassend die Herstellung des Adduktes aus einem Bisphenol und einem Phenol gemäß Anspruch 1, und die Gewinnung des Bisphenols aus dem Addukt aus dem Bisphenol und dem Phenol.

4. Verfahren zur Herstellung von Bisphenol A umfassend die Herstellung des Adduktes aus Bisphenol A und Phenol gemäß Anspruch 2 und die Gewinnung des Bisphenol A aus dem Addukt aus dem Bisphenol A und dem Phenol.

## Claims

1. A process for preparing crystals of an adduct of a bisphenol and a phenol comprising
a) the preparation of a solution containing bisphenol and phenol and
b) continuous performance of the crystallisation in one or more crystallisation devices which contain a crystallisation tank, a circulating pump and a cooler,
wherein the residence time of the liquid to be crystallised in the crystallisation tank is 2 to 12 hours and
wherein crystallisation is performed in 1 to 5 stages, each consisting of the crystallisation devices mentioned and
wherein the temperature in the crystallisation tank in the last crystallisation stage is 40 to 70°C and wherein the concentration of bisphenol in the inflow to the first crystallisation stage is 15 to 40 wt.% and
wherein, in the outflow from the last crystallisation stage, the concentration of bisphenol in dissolved form in the mother liquor is 10 to 20 wt.% and the proportion of crystallised solids in crystals of the adduct of a bisphenol and a phenol is 20 to 30 wt.%, **characterised in that** the specific pumping energy is at most 150 watts/m³ of suspension and the liquid flows from above downwards through the circulation cooler at a rate of 1 to 5 m/sec.

2. A process according to Claim 1, wherein the bisphenol is bisphenol A and wherein the phenol is unsubstituted phenol.

3. A process for preparing a bisphenol comprising preparing the adduct of a bisphenol and a phenol in accordance with Claim 1 and recovering the bisphenol from the adduct of the bisphenol and the phenol.

4. A process for preparing bisphenol A comprising preparing the adduct of bisphenol A and phenol in accordance with Claim 2 and recovering the bisphenol A from the adduct of bisphenol A and phenol.

## Revendications

1. Procédé de préparation de cristaux d'un adduit d'un bisphénol et d'un phénol comprenant:
a) la fourniture d'une solution contenant du bisphénol et du phénol et
b) l'exécution continue de la cristallisation dans un ou plusieurs dispositifs de cristallisation, qui contiennent un cristallisoir, une pompe de circulation et un refroidisseur,
le temps de séjour du liquide à cristalliser dans les cristallisoirs allant de 2 à 12 heures et
la cristallisation étant effectuée en 1 à 5 étapes composées respectivement desdits dispositifs de cristallisation et
la température dans le cristallisoir de la dernière étape de cristallisation allant de 40 à 70°C et
le taux de bisphénol dans l'alimentation de la première étape de cristallisation allant de 15 à 40% en poids, le taux de bisphénol sous forme dissoute dans la lessive-mère s'élevant à 10 - 20% en poids à la sortie de la dernière étape de cristallisation et le taux de matières solides cristallisées en cristaux de l'adduit d'un bisphénol et d'un phénol s'élevant à 20 - 30% en poids, **caractérisé en ce que** l'énergie de pompage spécifique s'élève au maximum à 150 Watt/m³ de suspension et le refroidisseur à circulation est parcouru de haut en bas à une vitesse de 1 à 5 m/s.

2. Procédé selon la revendication 1, le bisphénol étant du bisphénol A et le phénol étant un phénol non substitué.

3. Procédé de préparation d'un bisphénol comprenant la préparation de l'adduit à partir d'un bisphénol et d'un phénol selon la revendication 1 et l'extraction du bisphénol à partir de l'adduit de bisphénol et de phénol.

4. Procédé de préparation de bisphénol A comprenant la préparation de l'adduit de bisphénol A et de phénol selon la revendication 2 et l'extraction de bisphénol A à partir de l'adduit de bisphénol A et de phénol.
